# EUROPEAN PATENT APPLICATION

(11) **EP 4 317 169 A1**
(43) Date of publication of application: **07.02.2024**
(21) Application number: 22774718.5
(22) Date of filing: 07.02.2022
(51) Int. Cl.: C07K 1/14, C12N 1/21, C12N 15/31, C12N 15/70, C12P 21/00

(54) **PRODUCTION METHOD FOR FOREIGN PROTEIN USING ESCHERICHIA COLI**

(30) Priority: 22.03.2021 JP 2021046808
(71) Applicant: Kaneka Corporation, Osaka-shi, Osaka 530-8288 (JP)
(72) Inventor: YAMAGIWA Raika, Takasago-shi, Hyogo 676-8688 (JP); TOKUNAGA Tomohisa, Takasago-shi, Hyogo 676-8688 (JP); NISHIYAMA Tozo, Takasago-shi, Hyogo 676-8688 (JP); MINAKUCHI Kazunobu, Takasago-shi, Hyogo 676-8688 (JP)
(74) Representative: Hoffmann Eitle
(86) International application number: PCT/JP2022/004680
(87) International publication number: WO 2022/201917

(57) **Abstract**

This invention is intended to efficiently extract and purify a target foreign protein expressed with the use of an *E. coli* strain from the periplasm. This invention provides a method for producing a protein comprising the steps (a) to (c) below: (a) a step of introducing a gene encoding a target foreign protein into *E. coli* having modification in a gene associated with maintenance of the outer membrane structure; (b) a step of culturing the *E. coli* of (a); and (c) a step of recovering a target foreign protein from the periplasmic fraction of the microbial cell after culture.

## Description

### Technical Field

The present invention relates to a method for producing a foreign protein using *E. coli.*

### Background Art

Production of a recombinant protein via a genetic recombination technique involves the use of a wide variety of hosts. Examples of hosts include an animal cell, such as a Chinese hamster ovary (CHO) cell, and a microorganism, such as *E. coli* or yeast. Use of a microorganism is advantageous since a target protein can be produced within a shorter period of time at lower cost, compared with the use of an animal cell. In particular, *E. coli* is one of the most-researched microorganisms, *E. coli* grows within a short period of time and is easy to handle, and, accordingly, *E. coli* is frequently used in protein production.

When a protein is to be produced using *E. coli,* in general, a protein is produced in a cell. Accordingly, it is necessary to separate a culture medium into a microbial cell and a culture supernatant therein after fermentation culture and then to carry out periplasmic extraction, cell disruption, recovery of an inclusion body, or the like. When cell disruption is to be performed, in particular, it is necessary to purify a target protein from a wide variety and a large quantity of host-derived substances. Such post-treatment processes significantly influence a production cost of the target protein.

In the case of a foreign protein, a method in which the content in the periplasm is selectively released without releasing the content in the cytoplasm may be employed, so that contamination of a host-derived protein can be reduced. Thus, in this case, a process for purification of a foreign protein is easier than a process for purification in the case from the cytoplasm. In addition, because the periplasm is an oxidizing environment, it is known that expression of a foreign protein in the periplasm would cause formation of intramolecular/intermolecular disulfide bonds and expression of foreign protein functions caused thereby. Under the above circumstances, a method in which a foreign protein is expressed in the periplasm is extensively employed in protein production (e.g., Patent Document 1 and Non-Patent Document 1).

### Prior Art Documents

### Patent Documents

Patent Document 1: JP H08-242879 A (1996)

### Non-Patent Documents

Non-Patent Document 1: C. Schimek et al., Biotechnol. Progress, 36: e2999, 2020

### Summary of the Invention

### Objects to Be Attained by the Invention

As described above, a method in which a target foreign protein is expressed in *E. coli* and the foreign protein is extracted from the *E. coli* periplasm has been extensively employed. However, the extraction efficiency of the target foreign protein is known to vary depending on the molecular weight, the expression level, and other conditions of the foreign protein to be expressed. Accordingly, a method for efficiently obtaining a foreign protein from *E. coli* regardless of the molecular weight or other conditions of such foreign protein is desired.

The present invention provides a method for producing a protein using *E. coli* in which a target foreign protein is localized in the *E. coli* periplasm and the protein can then be efficiently extracted and purified.

### Means for Attaining the Objects

The present inventors have conducted concentrated studies, and, as a result, discovered that the use of the *E. coli* strain having modification of a gene associated with maintenance of the outer membrane structure would enhance an efficiency for extraction of an expressed foreign protein localized in the periplasm. This has led to the completion of the present invention.

Specifically, the present description provides the invention described below.
(1) A method for producing a protein comprising the steps (a) to (c) below:
   (a) a step of introducing a gene encoding a target foreign protein into *E. coli* having modification in a gene associated with maintenance of the outer membrane structure;
   (b) a step of culturing the *E. coli* of (a); and
   (c) a step of recovering a target foreign protein from the periplasmic fraction of the microbial cell after culture.
(2) The method according to (1), wherein the gene associated with maintenance of the outer membrane structure is at least one gene selected from the group consisting of *pal, lpp, ompA, tolA, mepS, nlpI, arcA, bamD, cyoA, dppA, ecnB, mrcA, mrcB, oppA,* and *slyB.*
(3) The method according to (1) or (2), wherein two or more genes of the *E. coli* selected from the group consisting of *pal, lpp, ompA, tolA, mepS, nlpI, arcA, bamD, cyoA, dppA, ecnB, mrcA, mrcB, oppA,* and *slyB* are modified.
(4) The method according to (1) or (2), wherein at least one gene of the *E. coli* selected from the group consisting of *pal, lpp, ompA,* and *tolA* is modified.
(5) The method according to any of (1) to (4), wherein the modification is complete deletion.
(6) The method according to any of (1) to (4), wherein the modification is partial mutation.
(7) The method according to (6), wherein the modification is partial mutation of a signal sequence.
(8) The method according to (6), wherein the modification is partial mutation of a structural gene.
(9) The method according to any of (1) to (8), wherein the *E. coli* is derived from the B strain or the K12 strain.
(10) A method for preparing a host *E. coli* for protein production comprising the steps (i) and (ii):
   (i) a step of modifying a gene of *E. coli* associated with maintenance of the outer membrane structure; and
   (ii) a step of introducing a gene encoding a target foreign protein into the genetically-modified *E. coli* obtained in step (i).
(11) The method according to (10), wherein the *E. coli* expresses a target foreign protein and the target foreign protein is accumulated in the periplasm of the *E. coli.*

This description includes part or all of the content as disclosed in the description and/or drawings of Japanese Patent Application No. 2021-046808, which is a priority document of the present application.

### Effects of the Invention

The present invention can provide a method for producing a protein using *E. coli* in which a target foreign protein is localized in the *E. coli* periplasm and the protein can then be efficiently extracted and purified.

### Embodiments of the Invention

### 1. A method for producing a protein

The method for producing a protein according to the present invention comprises the steps (a) to (c) below:
(a) a step of introducing a gene encoding a target foreign protein into *E. coli* having modification in a gene associated with maintenance of the outer membrane structure;
(b) a step of culturing the *E. coli* of (a); and
(c) a step of recovering a target foreign protein from the periplasmic fraction of the microbial cell after culture.

The method according to the present invention has the characteristics described above, and the method thus enables efficient recovery of an expressed target foreign protein localized in the *E. coli* periplasm.

The term "foreign protein" used herein refers to a protein encoded by a gene incorporated from the outside of the host cell, and not generally expressed by the host cell which is not transformed.

### 1-1. E. coli strain

An *E. coli* strain used in the method for producing a protein according to the present invention (hereafter, the method may be referred to as "the method of the present invention") is a genetically-modified strain, a parent *E. coli* strain thereof is not particularly limited, and any known *E. coli* strain can be used. In particular, the B strain, a strain derived from the B strain, the K 12 strain, or a strain derived from the K12 strain can be preferably used. Alternatively, the B-K12 hybrid strain, the HB101 strain, can be used. Examples of known strains derived from the B strain include the BL21 strain and the REL606 strain. Examples of known strains derived from the K12 strain include the W3110 strain, the DH10B strain, the BW25113 strain, the DH5α strain, the MG1655 strain, the JM109 strain, and the RV308 strain.

### 1-2. Gene associated with outer membrane formation

An *E. coli* strain used in the method of the present invention is a genetically-modified strain in which one or more genes associated with maintenance of the outer membrane structure are modified.

The term "gene associated with outer membrane formation" used herein refers to a gene of *E. coli* associated with maintenance of the outer membrane structure. More specifically, the term "gene associated with outer membrane formation" refers to an *E. coli* gene associated with expression of a protein associated with maintenance of the outer membrane structure of *E. coli;* i.e., a nucleic acid (DNA or RNA, with DNA being preferable) of *E. coli.* A gene associated with outer membrane formation is not particularly limited, provided that such gene is associated with maintenance of the outer membrane structure. For example, such gene is preferably the *pal, lpp, ompA, tolA, mepS, nlpI, areA, bamD, cyoA, dppA, ecnB, mrcA, mrcB, oppA,* or *slyB* gene, more preferably the *pal, lpp, ompA,* or *tolA* gene, and further preferably the *pal* or *Ipp* gene. More specifically, such gene preferably comprises a nucleotide sequence having 60% or higher, 70% or higher, 80% or higher, 85% or higher, 90% or higher, 95% or higher, 96% or higher, 97% or higher, 98% or higher, or 99% or higher sequence identity to the nucleotide sequence as shown in any of SEQ ID NOs: 4 to 32.

The "sequence identity" of nucleotide sequences can be determined herein by a method, sequence analysis software, or the like well known in the art. Examples include the blastn program of the BLAST algorithm and the fasta program of the FASTA algorithm. In the present invention, the "sequence identity" of a nucleotide sequence to be evaluated to the nucleotide sequence X is a value determined by aligning the nucleotide sequence X and the nucleotide sequence to be evaluated, introducing gaps, according to need, so as to maximize the extent of nucleotide consistency therebetween, and determining a frequency (%) of identical nucleotides appearing at the same sites in the nucleotide sequences including the gaps.

Among the genes associated with outer membrane formation described above, *arcA* is a gene to express ArcA (aerobic respiration control). SEQ ID NO: 4 shows an example of a nucleotide sequence of a structural gene encoding ArcA *of E. coli.* SEQ ID NO: 5 shows a signal sequence *of arcA.*

*bamD* is a gene to express an outer membrane protein, BamD. SEQ ID NO: 6 shows an example of a nucleotide sequence of a structural gene encoding BamD of *E. coli.* SEQ ID NO: 7 shows a signal sequence of *bamD.*

*cyoA* is a gene to express a membrane protein, CyoA, constituting the respiratory system. SEQ ID NO: 8 shows an example of a nucleotide sequence of a structural gene encoding CyoA of *E. coli.* SEQ ID NO: 9 shows a signal sequence *of cyoA.*

*dppA* is a gene to express a dipeptide binding protein, DppA. SEQ ID NO: 10 shows an example of a nucleotide sequence of a structural gene encoding DppA of *E. coli.* SEQ ID NO: 11 shows a signal sequence *of dppA.*

*ecnB* is a gene to express a toxin lipoprotein, EcnB. SEQ ID NO: 12 shows an example of a nucleotide sequence of a structural gene encoding EcnB of *E. coli.* SEQ ID NO: 13 shows a signal sequence of *ecnB.*

*lpp* is a gene to express a major outer membrane lipoprotein, Lpp. SEQ ID NO: 14 shows an example of a nucleotide sequence of a structural gene encoding Lpp of *E. coli.* SEQ ID NO: 15 shows a signal sequence of *lpp.*

*mepS* is a gene to express peptidoglycan DD-endopeptidase/peptidoglycan LD-peptidase, MepS. SEQ ID NO: 16 shows an example of a nucleotide sequence of a structural gene encoding MepS of *E. coli.* SEQ ID NO: 17 shows a signal sequence of *mepS.*

*mrcA* is a gene to express peptidoglycan glycosyl transferase/peptidoglycan DD-transpeptidase, MrcA. SEQ ID NO: 18 shows an example of a nucleotide sequence of a structural gene encoding MrcA of *E. coli.* SEQ ID NO: 19 shows a signal sequence of *mrcA.*

*mrcB* is a gene to express peptidoglycan glycosyl transferase/peptidoglycan DD-transpeptidase, MrcB. SEQ ID NO: 20 shows an example of a nucleotide sequence of a structural gene encoding MrcB of *E. coli.* SEQ ID NO: 21 shows a signal sequence *of mrcB.*

*nlpI* is a gene to express a lipoprotein, NlpI. SEQ ID NO: 22 shows an example of a nucleotide sequence of a structural gene encoding NlpI of *E. coli.* SEQ ID NO: 23 shows a signal sequence of NlpI.

*ompA* is a gene to express an outer membrane protein, OmpA. SEQ ID NO: 24 shows an example of a nucleotide sequence of a structural gene encoding OmpA of *E. coli.* SEQ ID NO: 25 shows a signal sequence of OmpA.

*oppA* is a gene to express a periplasmic oligopeptide-binding protein, OppA. SEQ ID NO: 26 shows an example of a nucleotide sequence of a structural gene encoding OppA of *E. coli.* SEQ ID NO: 27 shows a signal sequence *of oppA.*

*pal* is a gene to express an outer membrane lipoprotein, Pal. SEQ ID NO: 28 shows an example of a nucleotide sequence of a structural gene encoding Pal of *E. coli.* SEQ ID NO: 29 shows a signal sequence of *pal.*

*slyB* is a gene to express peptidyl-prolyl cis-trans isomerase, SlyB. SEQ ID NO: 30 shows an example of a nucleotide sequence of a structural gene encoding SlyB of *E. coli.* SEQ ID NO: 31 shows a signal sequence of *slyB.*

*tolA* is a gene to express a protein constituting the Tol/Pal system, TolA. The Tol/Pal system is a complex of a plurality of proteins existing in Gram-negative bacteria, and it is reported to be associated with outer membrane invagination during cell division and maintenance of outer membrane structure. SEQ ID NO: 32 shows an example of a nucleotide sequence of a structural gene encoding TolA of *E. coli.*

### 1-3. E. coli strain having modification in the gene associated with outer membrane formation

The term "modification" of a gene refers to addition of an alteration in a nucleotide sequence to a gene existing in nature (such gene is referred to as a "wild-type" gene). The "modification" is not particularly limited, provided that a certain kind of alteration is added to the nucleotide sequence of the original gene. Examples thereof include complete deletion and partial mutation.

The genetically-modified *E. coli* strain used in the method of the present invention has genetic modification that alters expression of one or more genes associated with outer membrane formation. The term "genetic modification that alters expression of one or more genes associated with outer membrane formation" used herein refers to genetic modification that significantly alters activity of a protein encoded by the gene, in comparison with activity of the parent strain. In particular, genetic modification that attenuates activity of a protein encoded by the gene, compared with activity of the parent strain, is preferable, and such modification encompasses genetic modification that would completely quench the activity. When protein activity is attenuated, the expression levels of mRNA, which is a transcription product of the target gene, or a protein, which is a translation product thereof, are lowered, or mRNA, which is a transcription product of the target gene, or a protein, which is a translation product thereof, would not normally function as mRNA or a protein.

The term "disruption" of a gene used herein refers to deletion or damage, with "deletion" being preferable. Examples of the partial mutation include deletion, insertion, and substitution, with partial deletion or partial substitution being preferable. The term "partial disruption" refers to disruption of a part of a gene or protein. The term "partial substitution" refers to substitution of a part of a nucleotide sequence of a gene or an amino acid sequence of a protein with another sequence. In the present invention, partial substitution that causes missense mutation is preferable. Hereafter, "one or more genes associated with outer membrane formation" to be modified may also be simply referred to as a "target gene." A target gene may be a gene associated with outer membrane formation or two or more genes associated with outer membrane formation. Alternatively, a plurality of sites of a gene associated with outer membrane formation may be modified.

When a part of the coding region of the amino acid sequence of the protein encoded by the target gene is to be deleted as the partial disruption, any region, such as the N-terminal region, the internal region, the C-terminal region, or other regions may be deleted. The reading frames of the upstream and downstream sequences of the region to be deleted are preferably inconsistent. It is preferable to delete at least a part of the coding region and/or the expression control sequence of the amino acid sequence of the target gene in the genomic DNA, such as a region consisting of a number of nucleotides that is, for example, at least 50%, at least 60%, at least 70%, at least 80%, at least 90%, or at least 95% of the total number of the nucleotides constituting the coding region and/or the expression control sequence. Alternatively, 100% of the region can be deleted (complete deletion). For example, a genetically-modified *E. coli* strain lacking at least a region from the start codon to the stop codon of the target gene of genomic DNA may be used.

The term "expression control sequence" used herein is not particularly limited, provided that such nucleotide sequence can be associated with expression of the coding region, and the expression control sequence may be a signal sequence and/or an SD sequence (e.g., 5'-AGGAGGA-3'). The genetically-modified *E. coli* strain of the present invention encompasses a modified strain with complete deletion or partial mutation of a signal sequence and/or an SD sequence.

Other examples of modification of the target gene of genomic DNA in the *E. coli* strain include introduction of missense mutation, introduction of a stop codon (nonsense mutation), and introduction of frameshift mutation via addition or deletion of 1 or 2 nucleotides into or from the amino acid sequence coding region of the gene in genomic DNA.

Modification of the target gene of genomic DNA in the *E. coli* strain can be achieved by, for example, insertion of other sequence into the expression control sequence or the amino acid sequence coding region of the gene in genomic DNA. The other sequence may be inserted into any region of the gene. It is preferable that reading frames of upstream and downstream sequences of the site of insertion be inconsistent. While the "other sequence" is not particularly limited, an example thereof is a marker gene. Examples of marker genes include, but are not limited to, drug-resistant markers reacting with drugs, such as kanamycin, ampicillin, tetracycline, and chloramphenicol, and auxotrophic markers reacting with nutrients, such as leucine, histidine, lysine, methionine, arginine, tryptophan, and uracil.

The genetically-modified *E. coli* strain can be obtained by, for example, mutagenesis, gene recombination, gene expression control using RNAi, gene editing, or the like.

Mutagenesis can be performed via ultraviolet irradiation or via treatment using a common agent causing mutation, such as N-methyl-N'-nitro-N-nitrosoguanidine (MNNG), ethyl methanesulfonate (EMS), methyl methanesulfonate (MMS), or the like.

Gene recombination can be performed in accordance with a known technique (e.g., FEMS Microbiology Letters 165, 1998, 335-340, JOURNAL OF BACTERIOLOGY, Dec. 1995, p7171-7177, Curr. Genet., 1986,10 (8): 573-578, or WO 98/14600).

A method in which a gene associated with outer membrane formation of *E. coli* having partial mutation is prepared and integrated into the genome of an arbitrary *E. coli* strain can be preferably employed. Examples of methods for preparing a gene associated with outer membrane formation *of E. coli* having partial mutation include methods known to a person skilled in the art, such as overlap PCR and total synthesis using adequate oligonucleotide primers. The prepared gene associated with outer membrane formation of *E. coli* having partial mutation can be adequately integrated into a host cell in accordance with an adequate conventional method. Examples of methods include a method involving the use of the Red-recombinase system (Datsenko K.A. and Wanner B.L., 2000, Proc. Natl. Acad. Sci., U.S.A., 97 (12), 6640-6645) and a method based on the homologous recombination mechanism of a host cell (Link A. J. et al., 1997, J. Bacteriol., 179, 6228-6237). In particular, the method described in Link *et al.* by which a foreign gene other than the gene of the *E. coli* genome would not be integrated into the genome is preferable.

Examples of methods for preparing recombinant vectors of the gene associated with outer membrane formation having partial mutation include, but are not particularly limited to, ligation, In-fusion (Clontec), PCR, and total synthesis. When an *E. coli* host is used, for example, a recombinant vector can be introduced into the host cell by the calcium chloride method, electroporation, or other methods, although the methods are not limited thereto.

In order to increase the amounts of foreign protein production and secretion, coexpression of periplasmic chaperones, such as FkpA, Dsb, and SurA, may be combined in a strain with partial mutation.

Modification of a target gene in genomic DNA of an *E. coli* strain can be realized by substitution of the gene in genomic DNA of the *E. coli* strain with a deletion gene or a marker gene. The term "deletion gene" used herein refers to an inactive gene modified so as not to produce a protein that normally functions by deleting a part or the whole of the target gene. Examples of deletion genes include a linear DNA comprising an arbitrary sequence without functions of the target gene wherein the linear DNA comprises upstream and downstream sequences of the target sites of substitution (i.e., a part or the whole of the target gene) on genomic DNA at the both ends of the arbitrary sequence or wherein the linear DNA comprises upstream and downstream sequences of the target sites of substitution on genomic DNA which are directly connected to each other. An example of a marker gene is a linear DNA comprising a sequence of the marker gene and upstream and downstream sequences of the target sites of substitution (i.e., a part or the whole of the target gene) on genomic DNA at the both ends of the marker sequence. The *E. coli* strain may be transformed with the linear DNA to cause homologous recombination in regions upstream and downstream of the target site of substitution in genomic DNA of the host strain. Thus, the target site of substitution can be substituted with the sequence of the linear DNA in a single step. As a result of such substitution, the target gene can be deleted from genomic DNA of the *E. coli* strain.

A marker gene may be removed after the substitution, according to need. When a marker gene is to be removed, sequences for homologous recombination or flippase recognition target (FRT) sequences may be added to both ends of the marker gene, so as to efficiently remove the marker gene.

When the genetically-modified *E. coli* strain is a strain in which the *pal* gene has been modified, examples thereof include an *E. coli* strain with mutation of a gene (SEQ ID NO: 40) encoding Pal (comprising the amino acid sequence as shown in SEQ ID NO: 33) in the *E. coli* genome and an *E. coli* strain with mutation of a gene (SEQ ID NO: 42) encoding a signal peptide of Pal. A signal peptide to be subjected to mutation may be another signal peptide, such as a signal peptide of OmpA, OmpF, or Lpp.

Pal mutation is preferably partial mutation and particularly preferably partial deletion. Examples *of E. coli* strains comprising a gene encoding Pal with partial deletion include an E. *coli* strain with partial deletion of a gene (SEQ ID NO: 28) encoding Pal (comprising the amino acid sequence as shown in SEQ ID NO: 33) in the *E. coli* genome and an *E. coli* strain with partial deletion of a gene (SEQ ID NO: 29) encoding a signal peptide (SEQ ID NO: 34) of Pal. The site to be partially deleted from Pal may be the site described in, for example, Cascales E. and Lloubes R., 2004, Mol. Microbiol., 51 (3), 873-885.

A strain with partial mutation of Pal can be prepared by introducing a gene comprising a gene encoding Pal with partial mutation in a Pal-deficient strain lacking a Pal-encoding gene to express Pal with partial mutation. In the present invention, an *E. coli* strain with partial mutation of Pal (SEQ ID NO: 33) is preferably used, and the partial mutation is more preferably partial deletion.

The partial mutation of Pal is preferably partial mutation in at least one amino acid sequence selected from among an amino acid sequence of the signal peptide of Pal and amino acid sequences of positions 3 to 17, positions 19 to 121, and positions 123 to 152 of SEQ ID NO: 49.

Examples *of E. coli* strains with mutation of Pal include an *E. coli* strain comprising, in the genome, a gene (SEQ ID NO: 36) encoding Pal (SEQ ID NO: 35) lacking amino acids 3 to 17, an *E. coli* strain comprising, in the genome, a gene (SEQ ID NO: 38) encoding Pal (SEQ ID NO: 37) lacking amino acids 19 to 43, an *E. coli* strain comprising, in the genome, a gene (SEQ ID NO: 40) encoding Pal (SEQ ID NO: 39) lacking amino acids 44 to 62, an *E. coli* strain comprising, in the genome, a gene (SEQ ID NO: 42) encoding Pal (SEQ ID NO: 41) lacking amino acids 62 to 93, an *E. coli* strain comprising, in the genome, a gene (SEQ ID NO: 44) encoding Pal (SEQ ID NO: 43) lacking amino acids 94 to 121, an *E. coli* strain comprising, in the genome, a gene (SEQ ID NO: 46) encoding Pal (SEQ ID NO: 45) lacking amino acids 123 to 152, an *E. coli* strain comprising, in the genome, a gene (SEQ ID NO: 48) encoding Pal (SEQ ID NO: 47) lacking amino acids 126 to 129, and an *E. coli* strain comprising, in the genome, a gene (SEQ ID NO: 50) encoding Pal (SEQ ID NO: 49) lacking amino acids 144 to 147.

As the *E. coli* strain used in the method of the present invention, an *E. coli* strain comprising the pal gene having the nucleotide sequence as shown in any of SEQ ID NO: 36, 38, 40, 42, 44, 46, 48, or 50 is preferably used.

Pal with the partial mutation may comprise an amino acid sequence derived from the amino acid sequence as shown in any of SEQ ID NO: 35, 37, 39, 41, 43, 45, 47, or 49 by substitution, deletion, and/or addition of one or a plurality of amino acids. The term "one or a plurality of" refers to, for example, 1 to 80, preferably 1 to 70, preferably 1 to 60, preferably 1 to 50, preferably 1 to 40, preferably 1 to 30, preferably 1 to 20, preferably 1 to 15, preferably 1 to 10, preferably 1 to 5, preferably 1 to 4, preferably 1 to 3, preferably 1 to 2, or preferably 1.

Pal with the partial mutation may comprise an amino acid sequence having 60% or higher sequence identity to the amino acid sequence as shown in any of SEQ ID NO: 36, 38, 40, 42, 44, 46, 48, or 50. The "sequence identity" may be 70% or higher, 80% or higher, 85% or higher, 90% or higher, 95% or higher, 96% or higher, 97% or higher, 98% or higher, or 99% or higher.

The "sequence identity" of amino acid sequences can be determined herein by a method, sequence analysis software, or the like well known in the art. Examples include the blastp program of the BLAST algorithm and the fasta program of the FASTA algorithm. In the present invention, the "sequence identity" of an amino acid sequence to be evaluated to the amino acid sequence X is a value determined by aligning the amino acid sequence X and the amino acid sequence to be evaluated, introducing gaps, according to need, so as to maximize the extent of amino acid consistency therebetween, and determining a frequency (%) of identical amino acids appearing at the same sites in the amino acid sequences including the gaps.

Examples of genetically-modified *E. coli* strains with modification of the *lpp* gene include an *E. coli* strain with mutation of a gene (e.g., the nucleotide sequence as shown in SEQ ID NO: 14) encoding Lpp (e.g., Lpp comprising the amino acid sequence as shown in SEQ ID NO: 51) in the *E. coli* genome and an *E. coli* strain with mutation of a gene (e.g., the nucleotide sequence as shown in SEQ ID NO: 15) encoding a signal peptide of Lpp. A signal peptide to be subjected to mutation may be another signal peptide, such as a signal peptide of OmpA, OmpF, or Pal.

Examples of genetically-modified *E. coli* strains with modification of the *ompA* gene include an *E. coli* strain with mutation of a gene (e.g., the nucleotide sequence as shown in SEQ ID NO: 24) encoding OmpA (e.g., OmpA comprising the amino acid sequence as shown in SEQ ID NO: 52) in the *E. coli* genome and an *E. coli* strain with mutation of a gene (e.g., the nucleotide sequence as shown in SEQ ID NO: 36) encoding a signal peptide of OmpA. A signal peptide to be subjected to mutation may be another signal peptide, such as a signal peptide of Lpp, OmpF, or Pal.

An example of a genetically-modified *E. coli* strain with modification of the *tolA* gene is an *E. coli* strain with mutation of a gene (e.g., the nucleotide sequence as shown in SEQ ID NO: 32) encoding TolA (e.g., TolA comprising the amino acid sequence as shown in SEQ ID NO: 53) in the E. *coli* genome.

### 1-4. Step of gene introduction (Step (a))

The method of the present invention comprises (a) a step of introducing a gene encoding a target foreign protein into an *E. coli* with modification in the gene associated with maintenance of the outer membrane structure (hereafter, the step may be referred to as "Step (a)").

### 1-4-1. Expression vector

The method of the present invention comprises introducing a gene encoding a target foreign protein (a foreign protein) into the genetically-modified *E. coli* strain to produce a target foreign protein. In the method of the present invention, specifically, an expression vector comprising a gene encoding a target foreign protein is introduced into the genetically-modified *E. coli* strain.

The term "expression vector" used herein refers to an artificially constructed nucleic acid molecule that enables a gene in an expression cassette integrated into the expression vector to express in a transformed host cell. An expression vector can comprise, in addition to an expression cassette, a cloning site comprising at least one restriction enzyme recognition sequence, an overlap region to use, for example, the In-Fusion Cloning System (Clontec), a marker gene, such as a drug resistant gene, an autonomously replicating sequence, and the like. Examples of expression vectors include plasmid vectors and artificial chromosome. From the viewpoint of ease of preparation of a vector and transformation of an *E. coli* strain, use of a plasmid vector is preferable. Examples of plasmids include, but are not particularly limited to, known expression vectors, such as pBR322, pBR325, pUC118, pUC119, pUC18, pUC19, and pBluescript.

An "expression vector" may be composed of a promoter and a gene encoding a foreign protein, and it may comprise a terminator. Examples of promoters used include, but are not particularly limited to, promoters known to a person skilled in the art, such as lac promoter, tac promoter, ara promoter, tet promoter, and T7 promoter.

In the method of the present invention, it is necessary that a target foreign protein is transported from the *E. coli* cytoplasm to the periplasmic space. It is thus necessary that the periplasmic transport signal sequence be added to the 5'-terminal side of the gene encoding the foreign protein. Examples of periplasmic transport signals that exert functions in *E*. *coli* include, but are not particularly limited to, PelB, OmpA, PhoA, OmpF, and STII. In the case of a foreign protein that does not require a periplasmic transport signal for being transported to the periplasmic space, addition of a periplasmic transport signal is not necessary.

In the method of the present invention, a length of a nucleotide sequence of a gene (a foreign gene) encoding a foreign protein to be integrated into an expression vector is preferably about 200 to 4,500 bp, and particularly preferably about 500 to 2,000 bp, from the viewpoint of high stability of the expression vector and high efficiency for introduction of the expression vector into a host.

Examples of foreign proteins produced by the method of the present invention include enzymes derived from microorganisms and proteins derived from multicellular organisms, such as animals and plants. Examples thereof include, but are not limited to, phytase, protein A, protein G, protein L, amylase, glucosidase, cellulase, lipase, protease, glutaminase, peptidase, nuclease, oxidase, lactase, xylanase, trypsin, pectinase, isomerase, and fluorescent protein. Biopharmaceutical proteins are particularly preferable.

Examples of biopharmaceutical proteins include antibody fragments, such as VHH, scFv, and Fab, cytokines, growth factors, protein kinases, protein hormone, Fc-fusion proteins, and human serum albumin (HSA) fusion proteins.

Amino acids constituting the foreign protein may be naturally-occurring, unnatural, or modified amino acids. An amino acid sequence of a protein may be artificially modified or *de-novo* designed.

In the present invention, a drug resistant marker gene of a plasmid vector is not particularly limited in the *E. coli* strain provided in the present invention. Specific examples include a kanamycin resistant gene, an ampicillin resistant gene, a tetracycline resistant gene, and a chloramphenicol resistant gene, and the like can be selected based on resistance in media each containing kanamycin, ampicillin, tetracycline, and chloramphenicol.

### 1-4-2. Gene introduction

In the method of the present invention, the expression vector is introduced into the genetically-modified *E. coli* strain. A method for introducing an expression vector into an E. *coli* strain is not particularly limited, and an expression vector can be introduced into an *E. coli* strain by a method of gene introduction, such as electroporation, the calcium chloride method, the competent cell method, or the protoplast method.

### 1-5. Step of E. coli culture (Step (b))

The method of the present invention comprises a step of culturing the genetically-modified *E. coli* strain comprising a gene encoding a foreign protein obtained in Step (a) (hereafter, the step may be referred to as "Step (b)").

The genetically-modified *E. coli* strain can be cultured in an adequate medium. Culture may be performed by any of batch culture, fed-batch culture, or continuous culture. The medium may be either a synthetic or natural medium, provided that it contains nutrients necessary for the growth of the genetically-modified *E. coli* strain, such as carbon sources, nitrogen sources, inorganic salts, and vitamins.

Any carbon sources can be used, provided that carbon sources are assimilable by the genetically-modified *E. coli* strain. Examples of carbon sources include saccharides, such as glucose and fructose, alcohols, such as ethanol and glycerol, and organic acids, such as acetic acid.

Examples of nitrogen sources include ammonia, ammonium salt such as ammonium sulfate, a nitrogen compound such as amine, and a natural nitrogen source such as peptone.

Examples of inorganic salts include potassium phosphate, magnesium sulfate, sodium chloride, ferrous sulfate, and potassium carbonate.

Examples of vitamins include biotin and thiamine. In addition, substances required by the genetically-modified *E. coli* strain to grow (e.g., a required amino acid for an amino acid-requiring strain) can be added, according to need.

In the method of the present invention, a medium supplemented with peptone, yeast extract, and sodium chloride is preferably used. Peptone is preferably soybean peptone. A preferable example of a medium supplemented with peptone, yeast extract, and sodium chloride is 2× YT medium.

In the method of the present invention, culture conditions are not particularly limited. For example, culture is preferably carried out via shake culture or agitation culture. Culture is performed at 20°C to 50°C, preferably at 20°C to 42°C, and more preferably at 25°C to 33°C. A culture period is 3 hours to 5 days, and preferably 5 hours to 3 days.

In the method of the present invention, a nutrient or inducer can be supplied to a medium in shots or via feeding, according to need.

An adequate inducer is selected in accordance with a promoter to be used. In the case of direct induction by lac promotor or tac promoter or indirect induction by T7 promoter induced by lac promoter to express T7 RNA polymerase, for example, lactose or isopropyl-β-thiogalactopyranoside (IPTG) is used. When an IPTG is used as an inducer, protein expression can be induced with the addition of IPTG to the final concentration of 0.1 to 2.0 mM, and more preferably to 0.2 to 1.0 mM.

A timing for induction of foreign protein expression is not particularly limited, provided that a foreign protein is expressed and secreted in a culture supernatant. Such timing is preferably an early stage, a mid stage, or a later stage of the logarithmic growth phase, with the later stage of the logarithmic growth phase being particularly preferable.

### 1-6. Step of recovery of target protein (Step (c))

The method of the present invention comprises a step of recovering a target foreign protein from a periplasmic fraction of the microbial cell of the genetically-modified *E. coli* strain cultured in Step (b) (hereafter, the step may be referred to as "Step (c)").

### 1-6-1. Recovery of microbial cell

A microbial cell can be recovered from a culture medium by any conventional technique, such as centrifugation. In such a case, a culture supernatant may be recovered, and the target protein may be purified separately from the microbial cell, so as to further enhance a yield of a target protein.

### 1-6-2. Extraction of periplasmic fraction

A target protein is recovered from the recovered microbial cell. Recovery of a target protein necessitates extraction of a protein from the periplasmic fraction of the microbial cell without disrupting the microbial cell, so as to suppress contamination of a cytoplasmic protein. In Step (c), the protein can be extracted from the periplasmic fraction by any conventional technique.

As conventional techniques for extracting a protein from the *E. coli* periplasmic fraction, for example, the osmotic shock method, treatment with a chelate agent, treatment with a cell-wall degrading enzyme such as lysozyme, treatment with a weak chaotropic substance (e.g., arginine) (e.g., Patent Document 1), heating, treatment with a surfactant at low concentration, and freeze-thawing are known. In such techniques, conditions under which a protein can be extracted from the periplasmic fraction without exposing the cytoplasmic fraction to the outside of the cell because of excessive cell disruption are preferable from the viewpoint of the proportion of the target protein to impurities in the extract. Any conditions may be employed without particular limitation. Any of the techniques may be employed by itself or in adequate combination. In order to reduce a risk of cytoplasmic exposure because of *E. coli* cell disruction, chemical techniques, such as the cold osmotic shock method using the osmotic shock, a method involving the use of a chelate agent, and a method involving the use of a surfactant at low concentration, are more preferable to mechanical techniques, such as ultrasonic treatment or a method involving the use of a high-pressure homogenizer. In general, a yield of a target protein by such chemical techniques is known to be lower than that achieved by mechanical techniques. Because the genetically-modified *E. coli* strain is used in the method of the present invention, a target protein can be obtained at a higher yield even though the chemical technique is used.

The cold osmotic shock method is the most common technique used for extraction from the periplasmic fraction. For example, the cold osmotic shock method can be performed in the manner described below. That is, a microbial cell is suspended in a hypertonic sucrose solution prepared with the use of EDTA/Tris buffer, the cell is harvested, and the microbial cell is then suspended in ice-cold water. The cell is harvested again, and the supernatant is obtained as the periplasmic fraction. Before the microbial cell is suspended in a hypertonic solution, the microbial cell may be washed with Tris buffer. A cycle of suspension of the microbial cell in a hypertonic solution and harvest of the microbial cell from the hypertonic solution may be performed a plurality of times.

A method involving the use of a chelate agent can be performed, for example, in the manner described below. The microbial cell is suspended in a weakly basic Tris/EDTA buffer containing EDTA at relatively high concentration and incubation is performed at room temperature for 15 minutes to 6 hours. The cell is harvested again, and the supernatant is obtained as the periplasmic fraction. A cycle of suspension of the microbial cell in the Tris/EDTA buffer and harvest of the microbial cell from the Tris/EDTA buffer may be repeated. Such method may be referred to as the "Tris/EDTA method" herein.

An example of a method involving the use of a surfactant at low concentration is a method involving the use of bile salt, such as sodium cholate or sodium deoxycholate, that is less likely to cause protein denaturation. Specifically, such method can be performed, for example, in the manner described below. The microbial cell is suspended in a solution containing sodium deoxycholate at about 0.15% by weight, the cell suspension is subjected to a shake reaction at room temperature for about 30 minutes to 7 hours, the cell is harvested, and the supernatant is obtained as the periplasmic fraction. A cycle of suspension of the microbial cell in a sodium deoxycholate solution and harvest of the microbial cell from the sodium deoxycholate solution may be performed a plurality of times.

When the conventional method of periplasmic extraction described above is employed, a sufficient amount of proteins may not be included in the periplasmic fraction depending on, for example, a type of the target protein, when a common *E. coli* strain is used. The method of the present invention is advantageous because a larger quantity of target proteins can be included in the periplasmic fraction.

### 1-6-3. Protein purification

A target protein can be purified from the periplasmic fraction. Protein purification can be performed in accordance with any conventional method. Examples of methods that can be employed include ammonium sulfate precipitation, gel filtration, ion exchange chromatography, and affinity chromatography. Because cells are not disrupted in the method of the present invention, a target protein can be efficiently purified in the presence of a small amount of proteins other than the target protein.

A protein may be purified from the culture supernatant separately from or together with the periplasmic fraction. By recovering a protein from the culture supernatant, a protein yield can further be improved.

### 2. Method for producing host E. coli

The method for preparing a host *E. coli* strain used for producing the protein of the present invention (hereafter, the method may be referred to as "the method for preparing a host *E. coli* strain of the present invention") comprises the steps (i) and (ii):
(i) a step of modifying a gene of an *E. coli* strain associated with maintenance of the outer membrane structure; and
(ii) a step of introducing a gene encoding a target foreign protein into the genetically-modified *E. coli* strain obtained in (i).

The *E. coli* strain prepared by the method for preparing a host *E. coli* strain of the present invention expresses a target foreign protein by the gene introduced thereinto. In such a case, the target foreign protein is preferably accumulated in the periplasm of the *E. coli* strain.

### 2-1. Step of genetic modification of host E. coli (Step (i))

The method for preparing a host *E. coli* strain of the present invention comprises a step of modifying a gene of an *E. coli* strain associated with outer membrane formation (hereafter, the step may be referred to as "Step (i)"). The *E. coli* strain used in the method for preparing a host E. *coli* strain of the present invention is as described in the "1-1. *E. coli* strain" in the "1. A method for producing a protein" above. A gene associated with outer membrane formation to be modified is as described in "1-2. Gene associated with outer membrane formation" above. Specific methods, conditions, and other factors for modifying a gene of an *E. coli* strain associated with outer membrane formation are as described in "1-3. *E. coli* strain having modification in the gene associated with outer membrane formation" above.

### 2-2. Step of introduction of foreign gene (Step (ii))

The method for preparing a host *E. coli* strain of the present invention comprises a step of introducing a gene encoding a target foreign protein into the genetically-modified *E. coli* strain obtained in Step (i) (hereafter, the step may be referred to as "Step (ii)"). In Step (ii), the expression vector used for gene introduction, the method of introduction, and other conditions are as described in "1-4. Step of gene introduction (Step (a))" above.

### Examples

The present invention is described in greater detail with reference to the following examples, although the present invention is not limited to these examples.

The amino acid numbers used in the present invention are the numbers in a full-length protein comprising a signal sequence. Genetic recombination techniques employed in the present invention, such as the polymerase chain reaction (PCR), gene synthesis, isolation and purification of DNA, treatment with a restriction enzyme, cloning of modified DNA, and transformation, are known to a person skilled in the art. The following examples were performed in accordance with the procedures described in the manufacturers' instructions of reagents and apparatuses, unless otherwise specified.

In the following examples, PCR was carried out using Prime STAR HS DNA Polymerase (Takara Bio Inc.). The PCR product and the restriction enzyme reaction solution were purified using the QIAuick Gel Extraction Kit (QIAGEN). When a DNA fragment was to be prepared by a technique other than PCR, a common technique for gene synthesis was employed. A plasmid vector used for transformation was prepared by introducing the constructed vector into the *E. coli* DH5α competent cell (Takara Bio Inc.), culturing the obtained transformant, and amplifying the culture product. The plasmid was extracted from the plasmid-carrying strain using the FastGene Plasmid Mini Kit (NIPPON Genetics Co., Ltd.). The cloning vector used herein was prepared by modifying pTH18cs (National Institute of Genetics (NIG)) and comprised, as a marker, the tetracycline resistant gene. The target protein was expressed with the use of an expression vector having a lac repressor gene.

### (Example 1) Preparation of E. coli strain having modification in the gene associated with outer membrane formation

*E. coli* strains with complete deletion of *pal,* partial deletion of *pal,* and point mutation of *Ipp* were prepared with reference to the method of Link A. J. et al. (J. Bacteriol., 1997, 179, 6228-6237). In order to completely delete the *pal* gene, a DNA fragment comprising EcoRI and *SalI* cleavage regions at both ends and homology arms of approximately 500 bp upstream and downstream of the *pal* gene (SEQ ID NO: 1) was amplified. The DNA fragment and the cloning vector prepared above were cleaved with restriction enzymes EcoRI and *SalI* and the cleaved DNA fragments were ligated to each other. The plasmid (pΔ*pal*) thus prepared was introduced into the *E. coli* BL21 strain by the calcium chloride method, and the pΔ*pal*-carrying strain was selected using a tetracycline-containing medium. Deletion of the target gene via homologous recombination was confirmed in accordance with the method of Link A. J. *et al.,* and the resulting modified strain was designated as the Δ*pal* strain.

In the manner as described above, a DNA fragment comprising *Eco*RI and *Sal*I cleavage regions at both ends and homology arms of approximately 200 bp upstream and downstream of the target modified *pal* gene lacking a region of positions 39 to 63 of Pal (SEQ ID NO: 2) and a DNA fragment comprising homology arms of approximately 200 bp upstream and downstream of the target modified *Ipp* gene with Gly at position 14 of the Lpp signal sequence being substituted with Asp (SEQ ID NO: 3) were amplified to prepare cloning vectors (the vectors were designated as "ppal1" and "plpp 1"). ppal1 and plpp1 were introduced into the *E. coli* BL21 strain in the same manner as in the case of pΔ*pal,* and the modified strains were selected using a tetracycline-containing medium. Modification of the target gene via homologous recombination was confirmed, and the resulting modified strains were designated as the pal1 strain and the lpp1 strain, respectively.

### (Example 2) Measurement of efficiency for recovery of target protein from E. coli strain having modification in the gene associated with outer membrane formation

This example was performed to examine the efficiency for recovery of the target proteins from the wild-type strain and 3 types of the *E. coli* strains having modification in the genes associated with outer membrane formation, which were prepared in Example 1. In this example, the target protein; i.e., the anti-von Willebrand factor (vWF) VHH antibody, was produced in various *E. coli* strains, and the recovery efficiency was inspected. *E. coli* strains into which the expression vector comprising the gene encoding the anti-vWF VHH antibody (SEQ ID NO: 54) had been introduced via electroporation were cultured in 1 ml of a semisynthetic medium. The total of the anti-vWF nanobody (the target protein) included in a soluble fraction of the microbial cell was designated as the recovery rate of 100%, and the recovery efficiency of the target protein from the periplasmic fraction in each *E. coli* strain was calculated. Each fraction was prepared by the two techniques described below.

### (1) Cold osmotic shock method

A culture medium (250 µl) was centrifuged at 6,400× g for 10 minutes to separate the culture medium into the supernatant and the cells. The cells were suspended in 250 µl of a hypertonic solution (100 mM Tris/HCl, 500 mM sucrose, 0.5 mM EDTA, pH 8.0), allowed to stand at room temperature for 10 minutes, and centrifuged at 12,000× g for 5 minutes to recover the supernatant as a hypertonic fraction 1. The cells were resuspended in 250 µl of a hypertonic solution, washed, and centrifuged at 12,000× g for 5 minutes. The supernatant was recovered as a hypertonic fraction 2, and the cells were suspended in 250 µl of an ice-cold hypotonic solution (1 mM MgCh). The suspension was centrifuged at 12,000× g for 5 minutes, and the supernatant was recovered as a hypotonic fraction 1. The cells were suspended in 250 µl of a hypotonic solution, the cell suspension was centrifuged at 12,000× g for 5 minutes, and the supernatant was recovered as a hypotonic fraction 2. The cells were suspended in 250 µl of PBS, disrupted using an ultrasonic disruptor (UH-50, SMT Corporation) for 30 seconds, which was performed 2 times, and centrifuged at 12,000× g for 5 minutes to recover the supernatant as a cell lysate fraction 1. The cells were suspended in 250 µl of PBS, washed, and centrifuged at 12,000× g for 5 minutes to recover the supernatant as a cell lysate fraction 2.

### (2) Tris/EDTA method

In the same manner as in (1), 250 µl of a culture medium was centrifuged at 6,400× g for 10 minutes to separate the culture medium into the supernatant and the cells. The cells were suspended in 250 µl of a Tris/EDTA buffer (100 mM Tris/HCl, 10 mM EDTA, pH 7.4), allowed to stand at room temperature for 90 minutes, and centrifuged at 12,000× g for 5 minutes to recover the supernatant as a T/E fraction 1. The cells were resuspended in 250 µl of a Tris/EDTA buffer, washed, and centrifuged at 12,000× g for 5 minutes. The supernatant was recovered as a T/E fraction 2. The cells were suspended in 250 µl of PBS, disrupted using an ultrasonic disruptor (UH-50, SMT Corporation) for 30 seconds, which was performed 2 times, and centrifuged at 12,000× g for 5 minutes to recover the supernatant as a cell lysate fraction 1. The cells were suspended in 250 µl of PBS, washed, and centrifuged at 12,000× g for 5 minutes to recover the supernatant as a cell lysate fraction 2.

### Western blot analysis

To the fractions (6 µl each) recovered by the cold osmotic shock method and the Tris/EDTA method, 2 µl of 4× Laemmli Sample Buffer (Biorad) was added and mixed, the mixtures were heated at 95°C for 5 minutes, and 3 µl of aliquots were applied to polyacrylamide gel. Following SDS-PAGE, the resultants were blotted to the PVDF membrane, and the PVDF membrane was blocked with Blocking One (Nacalai tesque, INC.). Following washing with TBS-T, an antibody reaction was performed using the rabbit anti-humanized VHH polyclonal antibody and the goat anti-rabbit polyclonal antibody-HRP, detection was performed using the EzWestLumi plus (ATTO), and the band intensities were determined using ChemiDoc (Biorad) and Quantity One (Biorad). On the basis of band intensities, the recovery rate of each fraction was determined. In the cold osmotic shock method, a total of the hypertonic fractions 1 and 2 and the hypotonic fractions 1 and 2 was determined as the amount of recovery. In the Tris/EDTA method, a total of the T/E fractions 1 and 2 was determined as the amount of recovery. The proportion of the amount of recovery (the recovery efficiency) accounting for the total anti-vWF nanobody (the target protein) from the microbial cell including the amounts generated from the cell lysate fractions 1 and 2 was determined, and differences between the wild-type strain and the *E. coli* strain having modification in the gene associated with outer membrane formation were inspected (Table 1). As a result of comparison between the wild-type strain and the *E. coli* strain having modification in the gene associated with outer membrane formation, the recovery efficiency was found to be improved both by the cold osmotic shock method and by the Tris/EDTA method with the use of a strain having modification in *pal* or *lpp.*

**[Table 1]**

| Amount of recovery (%) of target protein (POI) in wild-type strain and *E. coli* strain having modification in gene associated with outer membrane formation | | |
|---|---|---|
| Strain | Extraction method | |
| | Cold osmotic shock method | Tris/EDTA method |
| BL21 strain | 17 | 32 |
| Δ*pal* strain | 45 | 64 |
| pal1 strain | 52 | 40 |
| lpp1 strain | 60 | 49 |

In all the strains, secretion of a part of the target protein was observed in the culture supernatant (data not shown). By further recovering the target protein from the culture supernatant, the amount of target protein recovery was found to be further improved.

### (Example 3) Measurement of efficiency for target protein recovery from the E. coli strain having modification in the gene associated with outer membrane formation using Keio collection

This example was performed to examine the efficiency for recovery of the target protein from the *E. coli* strain having modification in the gene associated with outer membrane formation using the Keio collection as a library of single-gene deletion mutants of *E. coli.* In this example, the target protein; i.e., the anti-vWF nanobody, was produced in *E*. *coli* BW25113 mutants of the Keio collection lacking *ompA, tolA, mepS, nlpI, arcA, cyoA, dppA, ecnB, mrcA, mrcB, oppA,* and *slyB,* and the recovery efficiency was inspected. The expression vector comprising the gene encoding the anti-vWF nanobody (SEQ ID NO: 54) was introduced via electroporation into mutants and cultured in 0.8 ml of a semisynthetic medium. The total of the anti-vWF nanobody (the target protein) included in a soluble fraction of the microbial cell of each mutant was designated as the recovery rate of 100%, and the recovery efficiency of the target protein recovered from the periplasmic fraction was calculated. Each fraction was prepared by the cold osmotic shock method under the same conditions as employed in Example 2. The anti-vWF nanobody of each fraction was subjected to Western blot analysis under the same conditions as in Example 2.

The recovery rate of the anti-vWF nanobody in each fraction was determined based on the band intensity. A total of the hypertonic fractions 1 and 2 and the hypotonic fractions 1 and 2 was designated as the amount of recovery. The proportion of the amount of recovery accounting for the total anti-vWF nanobody from the microbial cell including the amounts generated from the cell lysate fractions 1 and 2 was determined, and differences between the wild-type strain (BW25113 strain) and the *E. coli* strain having modification in the gene associated with outer membrane formation were inspected (Table 2). As a result of comparison between the wild-type strain and the *E. coli* strain having modification in the gene associated with outer membrane formation, the recovery efficiency from the *E. coli* strains having modification in the gene associated with outer membrane formation by the cold osmotic shock method was found to be improved with the use of strains lacking *ompA, tolA, mepS, nlpI, arcA, cyoA, dppA, ecnB, mrcA,* mrcB, *oppA,* and *slyB.*

**[Table 2]**

| Amount of recovery (%) of target protein (POI) in wild-type strain and *E. coli* strain having modification in gene associated with outer membrane formation subjected to the cold osmotic shock method | |
|---|---|
| Strain | Amount of recovery (%) |
| BW25113 strain | 39 |
| Δ*ompA* strain | 68 |
| Δ*tolA* strain | 75 |
| Δ*mepS* strain | 81 |
| Δ*nlpI* strain | 70 |
| Δ*arcA* strain | 61 |
| Δ*cyoA* strain | 54 |
| Δ*dppA* strain | 66 |
| Δ*ecnB* strain | 88 |
| Δ*mrcA* strain | 61 |
| Δ*mrcB* strain | 92 |
| Δ*oppA* strain | 71 |
| Δ*slyB* strain | 60 |

All publications, patents, and patent applications cited herein are incorporated herein by reference in their entirety.

## Claims

1. A method for producing a protein comprising the steps (a) to (c) below:
(a) a step of introducing a gene encoding a target foreign protein into *E. coli* having modification in a gene associated with maintenance of the outer membrane structure;
(b) a step of culturing the *E. coli* of (a); and
(c) a step of recovering a target foreign protein from the periplasmic fraction of the microbial cell after culture.

2. The method according to claim 1, wherein the gene associated with maintenance of the outer membrane structure is at least one gene selected from the group consisting of *pal, lpp, ompA, tolA, mepS, nlpI, arcA, bamD, cyoA, dppA, ecnB, mrcA, mrcB, oppA,* and *slyB.*

3. The method according to claim 1 or 2, wherein two or more genes of the E. *coli* selected from the group consisting of *pal, lpp, ompA, tolA, mepS, nlpI, arcA, bamD, cyoA, dppA, ecnB, mrcA, mrcB, oppA,* and *slyB* are modified.

4. The method according to claim 1 or 2, wherein at least one gene of the *E. coli* selected from the group consisting of *pal, lpp, ompA,* and *tolA* is modified.

5. The method according to any one of claims 1 to 4, wherein the modification is complete deletion.

6. The method according to any one of claims 1 to 4, wherein the modification is partial mutation.

7. The method according to claim 6, wherein the modification is partial mutation of a signal sequence.

8. The method according to claim 6, wherein the modification is partial mutation of a structural gene.

9. The method according to any one of claims 1 to 8, wherein the *E. coli* is derived from the B strain or the K12 strain.

10. A method for preparing a host *E. coli* for protein production comprising the steps (i) and (ii):
(i) a step of modifying a gene of *E. coli* associated with maintenance of the outer membrane structure; and
(ii) a step of introducing a gene encoding a target foreign protein into the genetically-modified *E. coli* obtained in the step (i).

11. The method according to claim 10, wherein the *E. coli* expresses a target foreign protein and the target foreign protein is accumulated in the periplasm of the *E. coli.*
